# EUROPEAN PATENT APPLICATION

(11) **EP 4 378 455 A1**
(43) Date of publication of application: **05.06.2024**
(21) Application number: 23212826.4
(22) Date of filing: 28.11.2023
(51) Int. Cl.: A61K 9/20, A61K 31/7048, A61P 3/10

(54) **A PHARMACEUTICAL FORMULATION COMPRISING EMPAGLIFLOZIN**

(30) Priority: 29.11.2022 TR 202201811
(71) Applicant: SANOVEL ILAC SANAYI VE TICARET A.S., Istanbul 34460 (TR)
(72) Inventor: SUNEL, Fatih, Istanbul (TR); PEHLIVAN AKALIN, Nur, Istanbul (TR); ARMUT, Merve, Istanbul (TR)
(74) Representative: Sevinç, Erkan

(57) **Abstract**

The present invention relates to a pharmaceutical formulation comprising Empagliflozin or a pharmaceutically acceptable salt thereof wherein Empagliflozin or pharmaceutically acceptable salt has a d (0.9) particle size between 1 and 5 µm. The present invention also relates to a simple, rapid, cost effective, time-saving and industrially convenient method of preparing the formulation.

## Description

### Field of the Invention

The present invention relates to a pharmaceutical formulation comprising Empagliflozin or a pharmaceutically acceptable salt thereof wherein Empagliflozin or pharmaceutically acceptable salt has a d (0.9) particle size between 1 and 5 µm. The present invention also relates to a simple, rapid, cost effective, time-saving and industrially convenient method of preparing the formulation.

### Background of the Invention

Empagliflozin is a potent inhibitor of sodium-glucose co-transporter type 2 (SGLT-2) and is therefore used to treat type 2 diabetes. It is currently approved for the treatment of type 2 diabetes and improvement of blood sugar control.

Empagliflozin is a white to yellowish non-hygroscopic crystalline solid, very slightly soluble in water (pH 1-7.4), slightly soluble in acetonitrile and ethanol, sparingly soluble in methanol, and practically insoluble in toluene. The chemical name of empagliflozin (1S)-1,5-anhydro-1-(4-chloro-3-{4-[(3S)-tetrahydrofuran-3-yloxy]benzyl}phenyl)-D- glucitol and its chemical structure is shown in the Formula- I.

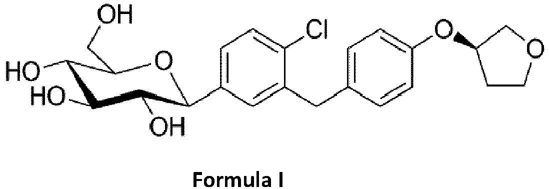

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

The first mention of the crystalline form of empagliflozin can be found in patent application WO 2006/117359. WO2006117360 discloses two other forms of empagliflozin which is described as Form I and Form II. Also, it disclosed methods for production thereof and synthesis of Empagliflozin.

EP 1730131 A1 discloses Empagliflozin, process for production thereof, its use and pharmaceutical composition thereof. The composition of a tablet is disclosed that comprises active ingredient in admixture with non-toxic pharmaceutically acceptable excipients, such as lactose, com starch, polyvinylpyrrolidone, magnesium stearate.

EP 2395968 A1 discloses formulations containing empagliflozin. Empagliflozin had a d (0.9) particle size between 10 µm and 100 µm. The active ingredient was used 25% or less of the weight of the pharmaceutical composition. The main object of the application is to provide a pharmaceutical composition containing empagliflozin that prevents or reduces adhesion during the manufacturing process.

WO2021250565 (A1) discloses a preparation of crystalline form of Empagliflozin and its particle size having coarser particle or D(0.5) equal or greater than 60 µm and D(0.9) equal or greater than 180 µm.

As evident from the prior art and the literature there are reported several pharmaceutical compositions comprising Empagliflozin. There is an existing and continual need for oral dosage formulations comprising Empagliflozin or a pharmaceutically acceptable salt thereof that have good dissolution profile, content uniformity, improved compressibility and manufactured by safe, effective, easy manufacturing methods.

We have found that empagliflozin having suitable particle sizes is very important for formulation. Especially, when empagliflozin or a pharmaceutically acceptable salt thereof has a d (0.9) particle size between 1 µm and 5 µm, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

### Detailed Description of the Invention

The main object of the present invention is to provide a pharmaceutical formulation comprising a therapeutically effective amount of Empagliflozin or a pharmaceutically acceptable salt thereof with the desired dissolution profile.

Another object of the present invention is to provide a pharmaceutical formulation comprising Empagliflozin or a pharmaceutically acceptable salt thereof with improved compressibility, flowability, homogeneity and high stability.

Another object of the present invention is to provide a pharmaceutical formulation comprising Empagliflozin or a pharmaceutically acceptable salt thereof prepared by simple, easy, time-saving and fast manufacturing methods.

The term "Dx" as used herein means that x% of the particles in a composition (based on volume) have a diameter of or below a specified d value. D (0.9)" or "d90" means that the size at which %90 by volume of the particles is finer. The volume mean particle size of the compound of the Empagliflozin is determined using Malvern Mastersizer 2000 laser diffraction particle size analyzer.

According to one embodiment of this invention, a pharmaceutical formulation comprises empagliflozin or a pharmaceutically acceptable salt thereof wherein empagliflozin has a d (0.9) particle size between 1 µm and 5 µm. These particle size helps to provide the dissolution properties and powder homogenization.

According to one embodiment, Empagliflozin or a pharmaceutically acceptable salt thereof has a d (0.9) particle size between 1 µm and 4 µm.

According to one embodiment of the present invention, the amount of Empagliflozin is present between 0.5% and %25.0 by weight in the total composition. Preferably, the amount of Empagliflozin is between 5.0% and 20.0% or 8.0% and 13.0% by weight in the total composition.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agent and the excipients.

According to this embodiment of the present invention, the formulation further comprises at least one pharmaceutically acceptable excipient is selected from the group comprising fillers, binders, disintegrants, lubricants or mixtures thereof.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

According to an embodiment of the present invention, the amount of fillers are 40.0% to 95.0% by weight in the total composition. Preferably, it is between 50.0% to 90.0% or between 60.0% and 90.0% by weight in the total formulation.

According to one embodiment of the present invention, the filler is microcrystalline cellulose.

According to one embodiment of the present invention, the filler is lactose monohydrate.

According to one embodiment of the present invention, the fillers are microcrystalline cellulose and lactose monohydrate.

Suitable binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 10.0% by weight of the total formulation. Preferably, the amount of binders is between 1.0% and 4.0% by weight of the total formulation.

According to this embodiment of the present invention, the binder is hydroxypropyl cellulose.

Suitable disintegrants are selected from the group comprising sodium starch glycolate, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

According to this embodiment of the present invention, the amount of disintegrants is between 1.0% and 10.0% by weight of the total formulation. Preferably, the amount of disintegrants is between 1.0% and 4.0% by weight of the total formulation.

According to this embodiment of the invention, the disintegrant is croscarmellose sodium.

Suitable glidants/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica, or mixtures thereof.

According to an embodiment of the present invention, the glidant/lubricant is anhydrous colloidal silicon dioxide or magnesium stearate or a mixture thereof. These excipients provide the flowability of the mixture. Especially, anhydrous colloidal silicon dioxide provides excellent flowability of powders during process.

According to an embodiment of the present invention a pharmaceutical formulation comprises empagliflozin or a pharmaceutically acceptable salt thereof wherein empagliflozin has a d (0.9) particle size between 1 µm and 5 µm and anhydrous colloidal silicon dioxide as lubricant.

According to an embodiment of the present invention, the pharmaceutical formulation is in the form of tablet or capsule.

According to an embodiment of the present invention, the pharmaceutical formulation comprises;
a) Empagliflozin
b) Microcrystalline Cellulose
c) Lactose monohydrate
d) HPC LF
e) Croscarmellose sodium
f) Anhydrous colloidal silicon dioxide
g) Magnesium stearate.

According to this embodiment, the coating material can include one or more following excipients of hydroxypropyl methylcellulose, hydroxypropyl cellulose, calcium Carbonate, isomalt, polyvinyl alcohol (PVA), polyethylene glycol (PEG), glycerin, talc, medium chain triglycerides, polyvinyl alcohol-polyethylene glycol copolymers, ^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to one embodiment of the present invention, a process for the preparation of the pharmaceutical formulation comprises;
- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and anhydrous colloidal silicon dioxide and then mixing,
- Adding magnesium stearate and then mixing,
- Compressing the mixture into the tablet,
- Coating the tablets with film coating agent.

### Example 1: Film coated tablet comprising empagliflozin

| **Ingredients** | **Amount (% by weight of the total formulation)** |
|---|---|
| Empagliflozin having d(90) particle size between 1 µm and 5 µm | 0.5-25.0 |
| Microcrystalline Cellulose PH101 | 10.0-50.0 |
| Lactose Monohydrate | 30.0-70.0 |
| HPC LF | 1.0 -10.0 |
| Croscarmellose sodium | 1.0-10.0 |
| Anhydrous colloidal silicon dioxide | 0.1-5.0 |
| Magnesium stearate | 0.1-5.0 |
| Coating | 0.5-4.0 |
| **TOTAL** | **100** |

### Example 2: Film coated tablet comprising empagliflozin

| **Ingredients** | **Amount (% by weight of the total formulation)** |
|---|---|
| Empagliflozin having d(90) particle size between 1 µm and 5 µm | 12.5 |
| Microcrystalline Cellulose PH101 | 25.0 |
| Lactose Monohydrate | 56.5 |
| HPC LF | 3.0 |
| Croscarmellose sodium | 2.0 |
| Anhydrous colloidal silicon dioxide | 0.5 |
| Magnesium stearate | 0.5 |
| Coating | 3.0 |
| **TOTAL** | **100** |

### A process for example 1 or 2;

- Mixing Empagliflozin, microcrystalline cellulose, lactose monohydrate and HPC,
- Granulating the mixture with water,
- Sieving the wet granule,
- Drying the wet granule in a fluidized bed,
- Sieving the mixture,
- Adding croscarmellose sodium and anhydrous colloidal silicon dioxide and then mixing,
- Adding magnesium stearate and then mixing,
- Compressing the mixture into the tablet,
- Coating the tablets with film coating agent.

### Film coating

| |
|---|
| Calcium Carbonate |
| Hydroxypropyl Methy Cellulose |
| Isomalt |
| Medium Chain Triglycerides |

### Example 3: A capsule comprising empagliflozin

| **Ingredients** | **Amount (% by weight of the total formulation)** |
|---|---|
| Empagliflozin having d(90) particle size between 1 µm and 5 µm | 0.5-20.0 |
| Fillers | 40.0 - 80.0 |
| Binders | 1.0 - 10.0 |
| Disintegrant | 1.0 - 10.0 |
| Glidants /lubricant | 1.0 - 10.0 |
| **TOTAL** | **100** |

## Claims

1. A pharmaceutical formulation comprising empagliflozin or a pharmaceutically acceptable salt thereof wherein empagliflozin has a d (0.9) particle size between 1 µm and 5 µm.

2. The pharmaceutical formulation according to claim 1, wherein Empagliflozin or a pharmaceutically acceptable salt thereof has a d (0.9) particle size between 1 µm and 4 µm.

3. The pharmaceutical formulation according to claim 1, wherein the amount of Empagliflozin is present between 0.5% and %25.0 by weight in the total composition.

4. The pharmaceutical formulation according to claim 1, wherein the tablet further comprises at least one pharmaceutically acceptable excipient is selected from the group comprising fillers, binders, disintegrants, glidants/lubricants or mixtures thereof.

5. The pharmaceutical formulation according to claim 4, wherein fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

6. The pharmaceutical formulation according to claim 5, wherein the filler is microcrystalline cellulose.

7. The pharmaceutical formulation according to claim 5, wherein the filler is lactose monohydrate.

8. The pharmaceutical formulation according to claim 4, wherein binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

9. The pharmaceutical formulation according to claim 8, wherein the binder is hydroxypropyl cellulose.

10. The pharmaceutical formulation according to claim 4, wherein disintegrants are selected from the group comprising sodium starch glycolate, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

11. The pharmaceutical formulation according to claim 10, wherein the disintegrant is croscarmellose sodium.

12. The pharmaceutical formulation according to claim 4, wherein glidants/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica, or mixtures thereof.

13. The pharmaceutical formulation according to claim 12, wherein the glidant/lubricant is anhydrous colloidal silicon dioxide or magnesium stearate or a mixture thereof.

14. The pharmaceutical formulation according to claim 1, wherein empagliflozin has a d (0.9) particle size between 1 µm and 5 µm and anhydrous colloidal silicon dioxide as lubricant.

15. A process for the preparation of the pharmaceutical formulation formulation comprises;
a) Mixing Empagliflozin, microcrystalline cellulose, lactose monohydrate and HPC,
b) Granulating the mixture with water,
c) Sieving the wet granule,
d) Drying the wet granule in a fluidized bed,
e) Sieving the mixture,
f) Adding croscarmellose sodium and anhydrous colloidal silicon dioxide and then mixing,
g) Adding magnesium stearate and then mixing,
h) Compressing the mixture into the tablet,
i) Coating the tablets with film coating agent.
